Europäisches Patentamt

(19) European Patent Office

Office européen des brevets

(11) Publication number: **0 233 620**
**B1**

(12) EUROPEAN PATENT SPECIFICATION

(45) Date of publication of the patent specification:
19.09.90

(21) Application number: **87102126.7**

(22) Date of filing: **14.02.87**

(51) Int. Cl.⁵: **B01J 20/32**, C07K 3/18,
A61K 35/16

(54) Use of an adsorbent for purification of blood coagulation factor VIII.

(30) Priority: **17.02.86  JP 32168/86**

(43) Date of publication of application:
**26.08.87 Bulletin 87/35**

(45) Publication of the grant of the patent:
**19.09.90 Bulletin 90/38**

(84) Designated Contracting States:
**AT CH DE FR GB LI**

(56) References cited:
**EP-A- 0 110 409**
**DE-A- 3 504 385**
**US-A- 4 397 841**

(73) Proprietor: **KANEGAFUCHI KAGAKU KOGYO**
**KABUSHIKI KAISHA, 2-4 Nakanoshima 3-chome, Kita-ku**
**Osaka-shi Osaka-fu(JP)**

(72) Inventor: **Youko, Nagano,**
**Moriyuki-Bunka 12 3-10 Kagoikedori 7-chome Chuo-ku,**
**Kobe-shi Hyogo-ken(JP)**
Inventor: **Nobutaka, Tani, 4-17-29, Fuminosato,**
**Abeno-ku, Osaka-shi Osaka-fu(JP)**

(74) Representative: **Türk, Gille, Hrabal, Brucknerstrasse 20,**
**D-4000 Düsseldorf 13(DE)**

**Description**

The present invention relates to the use of an adsorbent to separate a blood coagulation factor VIII from the body fluid.

Antihemophilic factor A is a synonym for blood coagulation factor VIII. It is an effective and common treatment for bleeding caused by hemophilia A to administer blood coagulation factor VIII which hemophilia A patients are deficient in. However, it is difficult to collect and purify blood coagulation factor VIII from human plasma since there exists only a very small amount of blood coagulation factor VIII in blood plasma and since it is unstable.

Cryoprecipitate and a factor VIII-concentrated pharmaceuticals are used to supply hemophilia patients with blood coagulation factor VIII at the present time.

While cryoprecipitate has such an advantage as a high yield of factor VIII, there are such disadvantages as an easily occurring-allergic reaction because of using a crude fraction of plasma, increase of fibrinogen concentration in plasma owing to a high fibronogen content in cryoprecipitate, and requirement of a large dosage owing to low concentration of factor VIII.

Though factor VIII concentrated pharmaceuticals is suitable for supplying hemophilia A patients since it does not have the above disadvantages, it has another problem that the yield of factor VIII is a very low value of about 20% when concentrated owing to a complicated process. That is to say, the factor VIII-concentrated pharmaceuticals is prepared from a factor VIII crude fraction such as cryoprecipitate or Cohn fraction I by complicated process comprising precipitation with Polyethylene glycol, precipitation with glycine and the like as shown in U.S. Patent No. 3 631 018.

EP-A 110 409 discloses an adsorbent consisting of a water insoluble porous gel having an exclusion limit lying within the range of from $3 \times 10^5$ to $1 \times 10^8$ and having sulfate group on at least a part of the surface thereof.

However, adsorbents which are practically suitable for purification of the factor VIII are not known because an adsorbent, which has been made a trial so far, has a poor adsorption selectivity and a low yield of the factor VIII adsorbed.

In accordance with the present invention, there can be provided the use of an adsorbent for blood coagulation factor VIII, which is a water-insoluble porous gel having an exclusion limit of from $8 \times 10^5$ to $1 \times 10^8$ having sulfate group on at least a part of its surface.

Fig. 1 is a graph showing concentrations of total protein, factor VIII and fibrinogen, and NaCl concentration in each fraction obtained in Example 6.

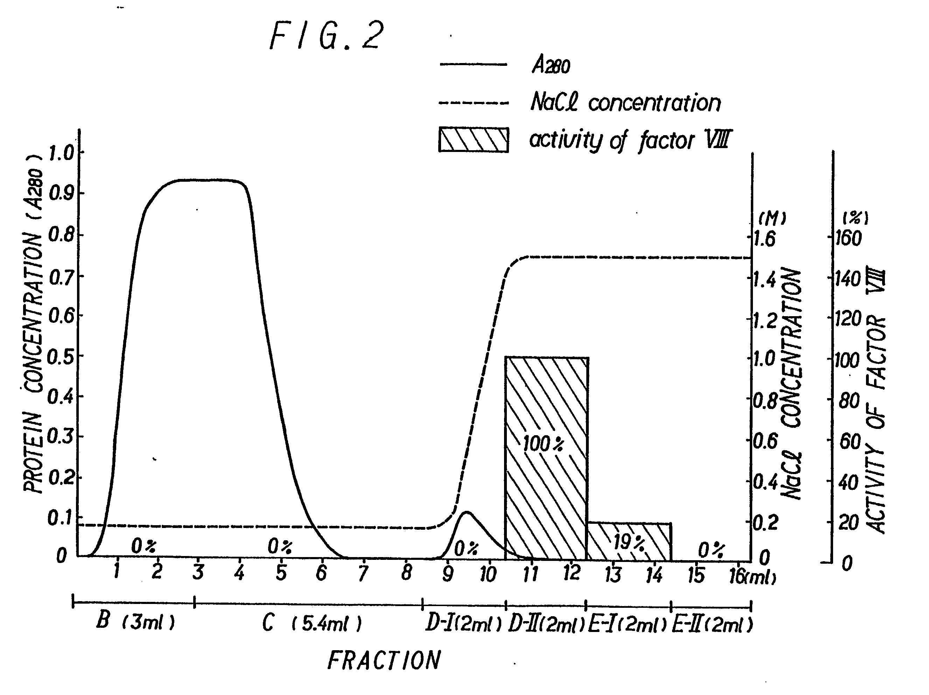

Fig. 2 is a graph showing concentrations of NaCl and protein, and activity of factor VIII in each fraction obtained in Example 7.

In the present invention, the term "body fluid" is blood, plasma, fractions thereof or any fluid components originated in a living body containing factor VIII.

It is preferable for a water-insoluble gel used in the present invention to have continuous large size pores. That is to say, it is required that the factor VIII, which is a macromolecule with the molecular weight of at least not less than $1 \times 10^6$, can easily permeate the gel to be adsorbed.

For measuring the pore size, there are various kinds of methods. Though mercury porosimetry is most frequently employed, it is difficult to apply to a hydrophilic gel. An exclusion limit is usually adopted as a mesure of the pore size of a hydrophilic gel.

The term "exclusion limit" in the present invention means, as described in the literatur such as "Jikken Kosoku Ekitai Chromatography (Experimental High Performance Liquid Chromatography)", Hiroyuki Hatano and Toshihiko Hanai, published by Kanbushiki Kaisha Kagaku Dojin, the minimum molecular weight of the molecule which cannot permeate into a pore, i.e. which is excluded, in a gel permeation chromatography. It is known that a value of an exclusion limit varies depending on the kind of the compound employed, among which exclusion limit values with such molecules as globular proteins, dextran and polyethylene glycol have been fully investigated. In the present invention, a value of an exclusion limit obtained from the globular proteins including virus, which are regarded as the most similar compounds to the factor VIII, is suitably employed.

As the result of an investigation, using various water-insoluble porous gels having various values of an exclusion limit, it is unexpectedly shown that a gel having an exclusion limit value of about $8 \times 10^5$, which is smaller than the molecular weight of factor VIII, can adsorb the factor VIII to some extent and that a gel having a larger pore size does not always exhibit an increased capacity of adsorption but, conversely, it is observed that an adsorption capacity of such gel decreases or proteins other than the factor VIII are likely to be adsorbed, which means there exists an optimum range of a pore size. That is, it is found that a water-insoluble porous gel having an exclusion limit of less than $8 \times 10^5$ can hardly adsorb the factor VIII and is not suited for practical use, whereas a water-insoluble porous gel having an exclusion limit of from $8 \times 10^5$ to $2 \times 10^6$, which is close on the molecular weight of factor VIII, can adsorb the factor VIII to some extent. Subsequently, it is observed that an amount of adsorbed factor VIII increases as an exclusion limit increases, and then reaches maximum, and it extremely decreases when an exclusion limit is over $1 \times 10^8$ because of too small surface area of the adsorbent.

Therefore, the exclusion limit of water-insoluble porous gel used in the present invention is preferably from $8 \times 10^5$ to $1 \times 10^8$, more preferably from $2 \times 10^6$ to $5 \times 10^7$.

With respect to a porous structure of a water-insoluble porous gel used in the present invention, a structure uniformly having pores at any part of the gel is more preferable than a structure having pores only on the surface of the gel. And it is preferred that a porosity of the gel used in the present invention is not less than 20%. A shape of a water-insoluble porous gel used in the present invention can be optionally selected from shapes such as particle, fiber, sheet and hollow fiber. When a water-insoluble porous gel with a shape of particle is used, the particle size is preferably from 1 to 5000 μm.

A water-insoluble porous gel used in the present invention can be organic or inorganic. It is preferred that the adsorption of blood components other than the desired factor VIII, so-called non-specific adsorption, is small.

Typical examples of water-insoluble porous gel used in the present invention are soft gels such as agarose, dextran and polyacrylamide, inorganic porous substances such as porous glass and porous silica gel, synthetic high molecular compounds such as polymethylmethacrylate, polyvinyl alcohol and styrene-divinylbenzene copolymer, porous polymer hard gels made of a natural high molecular compound such as cellulose. However, the present invention is not limited thereto.

Though a soft gel such as agarose has an advantage that its non-specific adsorption is low in comparison with a gel made of synthetic polymer or inorganic substance, a polymer hard gel is more suitable for preparation of plasma pharmaceuticals rather than a soft gel because adsorbing and desorbing operation using a polymer hard gel can be carried out with a high flow rate.

Since the porous cellulose gel has both above advantages belonging to a soft gel and to a hard gel and since sulfate group can be easily introduced into it, the porous cellulose gel is particularly suitable for the adsorbent of the present invention.

There are various methods to introduce sulfate group into a water-insoluble porous gel. Among them, a method of immobilizing a compound having sulfate group onto a water-insoluble porous gel and a method of introducing sulfate group by sulfation of hydroxyl group of a hydroxyl group-containing water-insoluble porous gel containing hydroxyl group directly by using a reagent such as clorosulfonic acid and a concentrated sulfuric acid are typical. In case of immobilizing a compound having sulfate group to a water-insoluble porous gel, it is preferred to have a covalent bond between the compound and the gel for a high stability.

For compounds having sulfate group, there are sulfuric esters of compounds having hydroxyl group, for instance, alcohol, sugar, polyhydric alcohol, carbohydrate and the like. Among them, compounds having a functional group by which the compound can be immobilized onto a water-insoluble porous gel besides sulfate group are preferable. Particularly, a polyhydric alcohol having sulfate group in part, especially a sulfuric ester of saccharide is preferable because it has both of sulfate group and a functional group necessary to the immobilization and it has both of high biocompatibility and high activity. Further, a sulfated polysaccharide which can be immobilized onto a water-insoluble porous gel is more preferable as a compound having sulfate group.

Typical examples of a compound having sulfate group are sulfuric esters of alcohols or polyhydric alcohols such as ethanolamine, ethylene glycol, glycerin, anisole, pentaerythritol, sorbitol, polyvinyl alcohol and polyhydroxyethyl methacrylate; sulfuric esters of sugars or carbohydrates such as glucose, xylose, threose, galactose, fucose, galactosamine, uronic acid, glucuronic acid and ascorbic acid; sulfuric esters of polysaccharides such as heparin, dextran sulfate, chondroitin sulfate, chondroitin polysulfate, heparan sulfate, keratan sulfate, xylan sulfate, charonin sulfate, cellulose sulfate, chitin sulfate, chitosan sulfate, pectin sulfate, inulin sulfate, alginine sulfate, glycogen sulfate, polylactose sulfate, carrageenan sulfate, sulfated starch, polyglucose sulfate, laminarin sulfate, galactan sulfate, levan sulfate and mepesulfate. However, the present invention is not limited thereto.

Sulfuric esters of polysaccharide having a low molecular weight of not more than $1 \times 10^5$ is preferable for hardly adsorbing fibrinogen and the like other than the factor VIII. Sulfuric esters of polysaccharides with sulfur content of from 5% to 20% is also preferable for a high adsorption activity.

It is preferable for an adsorbent to contain sulfate group, which are introduced by various methods, of from 0.1 μmol to 10 millimol/ml of the sulfate. A sulfate grup content of less than 0.1 μmol/ml causes insufficient adsorption capacity and a sulfate group content of more than 10 millimol/ml causes too much non-specific adsorption, especially fibrinogen adsorption for practical use. It is more preferable that sulfate group content is from 1 μmol to 50 μmol/ml of the adsorbent.

The factor VIII can be separated from a solution containing the factor VIII using the adsorbent of the present invention by following process. After the factor VIII is adsorbed by contacting the solution with the adsorbent, components not adsorbed are washed off, and then the factor VIII is eluted.

There are various methods to elute the adsorbed factor VIII, for instance, heating, changing pH and the like. Among them, a method of elution with an aqueous solution with a high ionic strength is preferable for simple treatment following the elution. In case that components other than factor VIII are adsorbed owing to the kind of adsorbent, factor VIII can be separated by changing the ionic strength, pH and the like continuously so-called gradient elution or by steps.

3

The present invention is more specifically described and explained by means of the following Examples. It is to be understood that the present invention is not limited to the Examples, and various changes and modifications might be made in the invention without departing from the spirit and scope thereof.

Example 1

Ten ml of a porous cellulose gel (CK gel A-3 made by Chisso Corporation, exclusion limit of globular proteins: $5 \times 10^7$, particle size: 45 to 105 μm) was dried by means of critical point drying method in ethanol. The resultant dried gel was suspended in 10 ml of pyridine sufficiently dehydrated and the suspension was cooled with ice, to which 2 ml of clorosulfonic acid was added by dropwise under stirring, the stirring being continued for 10 minutes after the dropwise addition was completed. After completion of the reaction, the gel was filtered and washed with pyridine and then water to give a cellulose gel on which surface sulfate group was introduced in an amount of 110 μmol per 1 ml of the gel.

Example 2

The procedures in Example 1 were repeated except for the reaction time of 60 minutes instead of 10 minutes after the dropwise addition of chlorosulfonic acid and the volume of chlorosulfonic acid of 3 ml instead of 2 ml to give a cellulose gel on which surface sulfate group in an amount of 750 μmol per 1 ml of the gel.

Example 3

Ten ml of a porous cellulose gel (Cellulofine GCL-2000 made by Chisso Corporation, exclusion limit of globular proteins: $3 \times 10^6$) was washed with water and then filtered under suction. To which 6 ml of dimethyl sulfoxide, 2.6 ml of 2N NaOH aqueous solution and 1.5 ml of epichlorohydrin were added. After completion of the reaction with stirring for 2 hours at 40°C, the resultant gel was filtered and washed with water to give a cellulose gel wherein epoxy group was introduced.

To the obtained gel, 6 ml of concentrated ammonium aqueous solution was added. After completion of the reaction for 2 hours at 40°C, an aminated cellulose gel was obtained.

There was added a solution that 4 g of sodium salt of dextran sulfate having a molecular weight of about 5,000 and having a sulfur content of 15% was dissolved in 8 ml of 0.1 M phosphate buffer solution of pH 8.0 to 2 g of the aminated cellulose gel. After completion of the reaction with shaking for 16 hours at a room temperature, 20 mg of NaCNBH₃ was added thereto and stirred for 30 minutes at room temperature. Then the mixture was heated for 4 hours at 40°C and the obtained gel was filtered and washed with water to give a cellulose gel wherein dextran sulfate was immobilized. The amount of dextran sulfate introduced was 3.4 mg per 1 ml of the gel.

Example 4

The procedures in Example 3 were repeated except that a dextran sulfate having a molecular weight of about $5 \times 10^5$ and having a sulfur content of 4.5% was used to give a cellulose gel wherein the amount of dextran sulfate introduced was 5.4 mg per 1 ml of the gel.

Reference Example

The procedures in Example 1 were repeated except that Cellulofine GC700 (made by Chisso Corporation, exclusion limit of globular proteins: $4 \times 10^5$, particle size: 45 to 105 μm) was used as a cellulose gel to give a cellulose gel on which surface sulfate group was introduced. The amount of sulfate group was 250 μmol per 1 ml of the gel.

Example 5

There was put 1 ml of the each gel prepared in Example 1–4 and Reference Example into a test tube and thereto 6 ml of citrated human plasma was added. The mixture was incubated for 1 hour at 37°C under stirring.

The activity of factor VIII, which was measured by means of APTT method, and the fibrinogen concentration in plasma after adsorption are shown on Table 1.

## Table 1

| Adsorbent (Example No. or Reference Example) | Activity of factor VIII (%) | Fibrinogen (mg/dℓ) |
|---|---|---|
| No adsorbent (Control) | 95 | 230 |
| Ex. 1 | 32 | 225 |
| Ex. 2 | 27 | 105 |
| Ex. 3 | 31 | 226 |
| Ex. 4 | 20 | 51 |
| Ref.Ex. | 82 | 218 |

As shown in Table 1, the adsorbents used in the present invention obtained in Examples 1 to 4 have higher adsorption capacity for the factor VIII in comparison with the adsorbent having an exclusion limit of $4 \times 10^5$ obtained in Reference Example. Further, the adsorbents used in the present invention obtained in Examples 1 to 3 have a good adsorption selectivity for the factor VIII in comparison with the adsorbent obtained by using dextran sulfate having the molecular weight of $5 \times 10^5$ and a sulfur content of 4.5% in Example 4. In addition, the adsorbent having sulfate group of 750 μmol/ml of the adsorbent obtained in Example 2 has lower adsorption selectivity for the factor VIII than ones obtained in Examples 1 and 3.

Example 6

One ml of adsorbent obtained in Example 1 was packed in a column made of polypropylene and 3 ml of human plasma was passed through the column. Then after 10 ml of saline was passed through the column to wash off components not adsorbed, solutions in which the NaCl concentration is continuously changed fromn 0.15 M to 2 M were passed through the column (gradient eluation). The eluate flowed out from the column was collected by a fraction collector.

The concentrations of total protein, factor VIII and fibrinogen in each fraction was measured. The concentration of factor VIII and fibrinogen was measured by means of Enzyme Immuno Assay.

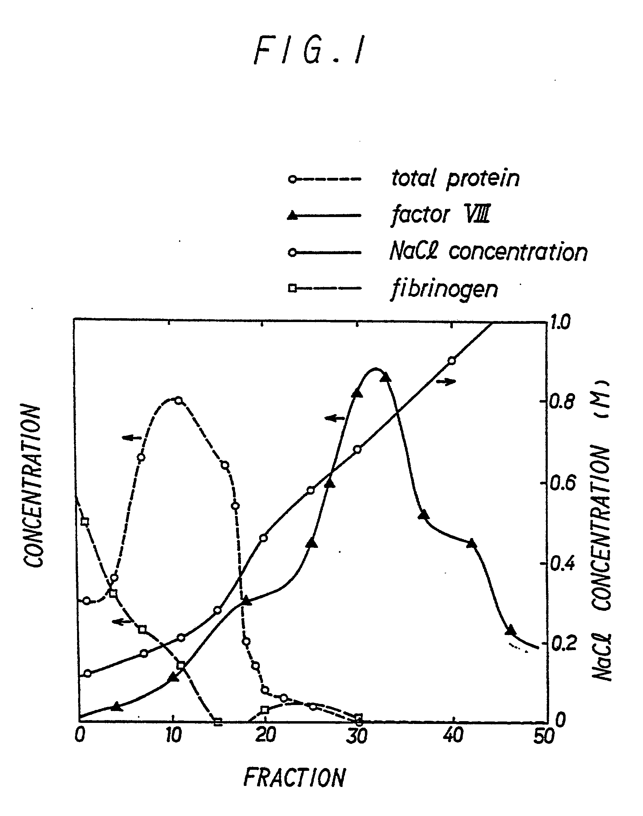

The results are shown in Figure 1. In Figure 1, concentrations of factor VIII and fibrinogen are shown as patterns of elution which do not indicate exact concentrations.

As shown in Figure 1, the blood coagulation factor VIII is eluted at a high NaCl concentration separated from other adsorbed proteins, especially fibrinogen.

Example 7

One ml of adsorbent prepared in Example 3 was packed in a column made of polypropylene and which is washed fully with 0.05 M tris(hydroxymethyl)aminomethanhydrochloric acid buffer solution of pH 7.4 containing 0.154 M NaCl. There was added 3 ml of a solution that KRYOBULIN which is a blood coagulation factor VIII-concentrated pharmaceuticals (made by Immuno AG) was dissolved in the above mentioned buffer solution so that the solution contains the factor VIII activity of 5 U/ml. After washing off components not adsorbed with 5 ml of the above butter, solutions in which the NaCl concentration is changed in two steps at 0.154 M and 1.5 M were passed through the column. The eluate flowed out from the column was collected to give several fractions.

The change of concentration of protein flowed out and activity of factor VIII, which was measured by means of APTT method, in each fraction B (3 ml), C (5.4 Ml), D-I (2 ml), D-II (2 ml), E-I (2 ml) and E-II (2 ml) are shown in Table 2.

As a result, factor VIII was adsorbed at almost 100% and collected by desorption with raising the NaCl concentration in a yield of 61%. And the specific activity (activity of factor VIII per 1 mg of protein) of the eluate was 145.12 which is about ten times of one in the original solution containing the factor VIII activity of 5 U/ml whose specific activity was 14.03.

In addition to the ingredients used in the Examples, other ingredients can be used in the Examples as set forth in the specification to obtain substantially the same results.

5

## Claims

1. Use of an adsorbent, which is a water-insoluble porous gel having an exclusion limit of globular proteins of from $8 \times 10^5$ to $1 \times 10^8$ and having sulfate group on at least a part of the surface thereof for the purification of blood coagulation factor VIII.

2. Use according to Claim 1, wherein said water-insoluble porous gel is composed of a compound having hydroxyl group.

3. Use according to Claim 2, wherein said sulfate group is introduced by sulfation of hydroxyl group of a hydroxyl group-containing water-insoluble porous gel.

4. Use according to Claim 1, wherein said sulfate group is introduced by immobilizing a compound having sulfate group onto said water-insoluble porous gel by covalent bond.

5. Use according to Claim 4, wherein said compound having sulfate group is a sulfated polysaccharide.

6. Use according to Claim 1, wherein the amount of said sulfate group is from 0.1 μmol to 10 millimol/ml of said adsorbent.

7. Use according to Claim 1, wherein the amount of said sulfate group is from 1 μmol to 500 μmol/ml of said adsorbent.

8. Use according to Claim 5, wherein the molecular weight of said sulfated polysaccharide is not more than $1 \times 10^5$.

## Patentansprüche

1. Verwendung eines Adsorbens, bei dem es sich um ein wasserunlösliches poröses Gel mit einer Ausschlußgrenze für kugelförmige Proteine von $8 \times 10^5$ bis $1 \times 10^8$ und mit einer Sulfatgruppe auf mindestens einem Teil der Oberfläche desselben handelt, für die Reinigung des Blutgerinnungsfaktors VIII.

2. Verwendung nach Anspruch 1, bei der das wasserunlösliche poröse Gel aus einer Verbindung mit einer Hydroxylgruppe besteht.

3. Verwendung nach Anspruch 2, bei der die Sulfatgruppe durch Sulfatierung der Hydroxylgruppe eines hydroxylgruppenhaltigen wasserunlöslichen porösen Gels eingeführt worden ist.

4. Verwendung nach Anspruch 1, bei der die Sulfatgruppe durch Immobilisierung einer Verbindung mit einer Sulfatgruppe auf dem wasserunlöslichen porösen Gel durch eine kovalente Bindung eingeführt worden ist.

5. Verwendung nach Anspruch 4, bei der die Verbindung mit einer Sulfatgruppe ein sulfatiertes Polysaccharid ist.

6. Verwendung nach Anspruch 1, bei der die Menge der Sulfatgruppe 0,1 μmol bis 10 mmol pro ml Adsorbens beträgt.

7. Verwendung nach Anspruch 1, bei der die Menge der Sulfatgruppe 1 μmol bis 500 μmol/ml Adsorbens beträgt.

8. Verwendung nach Anspruch 5, bei der das Molekulargewicht des sulfatierten Polysaccharids nicht mehr als $1 \times 10^5$ beträgt.

## Revendications

1. Utilisation d'un adsorbant, qui est un gel poreux, insoluble dans l'eau ayant une limite d'exclusion de protéines globulaires comprise entre $8 \times 10^5$ et $1 \times 10^8$ et ayant un groupe sulfate sur au moins une partie de sa surface pour purifier le facteur VIII de coagulation du sang.

2. Utilisation selon la revendication 1, dans laquelle ledit gel poreux insoluble dans l'eau comprend un composé ayant un groupe hydroxyle.

3. Utilisation selon la revendication 2, dans laquelle on introduit ledit groupe sulfate par sulfatation du groupe hydroxyle d'un gel poreux insoluble dans l'eau contenant un groupe hydroxyle.

4. Utilisation selon la revendication 1, dans laquelle on introduit ledit groupe sulfate en immobilisant un composé ayant un groupe sulfate par liaison covalente sur le gel poreux insoluble dans l'eau.

5. Utilisation selon la revendication 4, dans laquelle ledit composé ayant un groupe sulfate est un polysaccharide sulfaté.

6. Utilisation selon la revendication 1, dans laquelle la quantité dudit groupe sulfate est comprise entre 0,1 μmole et 10 millimoles/ml dudit adsorbant.

7. Utilisation selon la revendication 1 dans laquelle la quantité dudit groupe sulfate est comprise entre 1 μmole et 500 μmole/ml dudit adsorbant.

8. Utilisation selon la revendication 5, dans laquelle la masse moléculaire dudit polysaccaride sulfaté ne dépasse pas $1 \times 10^5$.

FIG.1

EP 0 233 620 B1

FIG. 2

——— $A_{280}$

- - - - - NaCl concentration

▨▨▨ activity of factor VIII

B (3ml)    C (5.4ml)    D-I(2ml) D-II(2ml) E-I(2ml) E-II(2ml)

FRACTION

EP 0 233 620 B1